# EUROPEAN PATENT APPLICATION

(11) **EP 1 647 403 A1**
(43) Date of publication of application: **19.04.2006**
(21) Application number: 05256028.1
(22) Date of filing: 27.09.2005
(51) Int. Cl.: B41J 2/14

(54) **Thermally efficient drop generator**

(30) Priority: 15.10.2004 US 619196 P; 02.03.2005 US 70915
(71) Applicant: Hewlett-Packard Development Company, L.P., Houston, TX 77070 (US)
(72) Inventor: Alley, Rodney L., Corvallis, OR 97330 (US); Yang, Xiaofeng, Corvallis, OR 97330 (US); Trueba, Kenneth E., Philomath, OR 97370 (US)
(74) Representative: Jackson, Richard Eric

(57) **Abstract**

A drop ejection device for use in a handheld inhaler is fabricated with a thin passivation layer (220) and thin or no metal anti-cavitation (230) layers above underlying heat transducers (212) to provide protection for the heat transducers. A control system (26) energizes selected heat transducers (212) to heat fluid in the chambers(170), vaporizing the fluid, which is ejected through the orifices (162) in small droplets.

## Description

### Technical Field

This invention relates to a thermal drop generator apparatus capable of generation of aerosolized droplets of liquid.

### Background of the Invention

Medications are often delivered to patients in the form of inhaled aerosols―gaseous suspensions of very fine liquid or solid particles in which medications are entrained. So-called pulmonary delivery of medication is in many instances a very efficient manner of delivering biological and chemical substances to the patient's bloodstream. Pulmonary delivery is especially efficient when the medication is delivered with a digitally controlled device such as a "metered dose inhaler" ("MDI") or other type of inhaler that incorporates ejector heads that are suitable for creating aerosols having very small droplet size. Such inhalers are often used to deliver asthma medications directly into a patient's lungs where the medications have a rapid anti-inflammatory effect. MDIs may also be used for systemic delivery of medication where the aerosolized droplets of medication are delivered deep into the lung tissue where the medication is rapidly absorbed into the patient's blood stream.

The most effective pulmonary drug delivery is accomplished when the medication is delivered in very small, aerosolized droplet directly to the alveoli―the tiny air sacs in the innermost lung tissue known as the alveolar epithelium―because the medication is transferred into the patient's bloodstream very rapidly.

Thermal-type drop generators may be used to generate aerosolized medications having desired small drop sizes. Such drop generators typically incorporate drop generator heads that have dielectric and metal layers interposed between the fluid medication and resistor heating elements that heat, and thus vaporize the fluid to eject it from nozzles. The dielectric and metal passivation and anticavitation layers provide necessary mechanical and electrical protection for the resistive layer. When the power to the resistor is turned off, the bubbles generated during operation collapse and may cause mechanical damage. If the passivation layer is compromised, fluid makes contact with the resistor heating elements, resulting in various types of damage such as corrosion. Despite the need for the protection afforded by passivation and anticavitation layers, significant energy is expended in heating the layers so that the medication may be vaporized. That is, energy from the resistive heating elements necessarily must be directed to the passivation and anticavitation layers rather than directly to the medium that is intended to be heated, the fluid medicament. This can lead to several concerns, including an increase in the power input requirements for such drop generators, even to the point where it is impractical to power the drop generator with a battery, and incidental heating of the drop generator during operation to the point where residual heating causes the fluid medication to boil even when the power is off.

There is a need therefore for a drop generator head having increased thermal efficiency.

### Brief Description of the Drawings

Apparatus and methods for carrying out the invention are described in detail below. Other advantages and features of the present invention will become clear upon review of the following portions of this specification and the drawings.
Fig. 1 is a schematic side elevation view of a metered dose inhaler apparatus, which is one example of a medication delivery apparatus in which the illustrated embodiment of drop generator head of the present invention is incorporated.
Figs. 2 through 6 illustrate a variety of nozzle arrangements that are appropriate for use in the drop generator head of the present invention. Specifically,

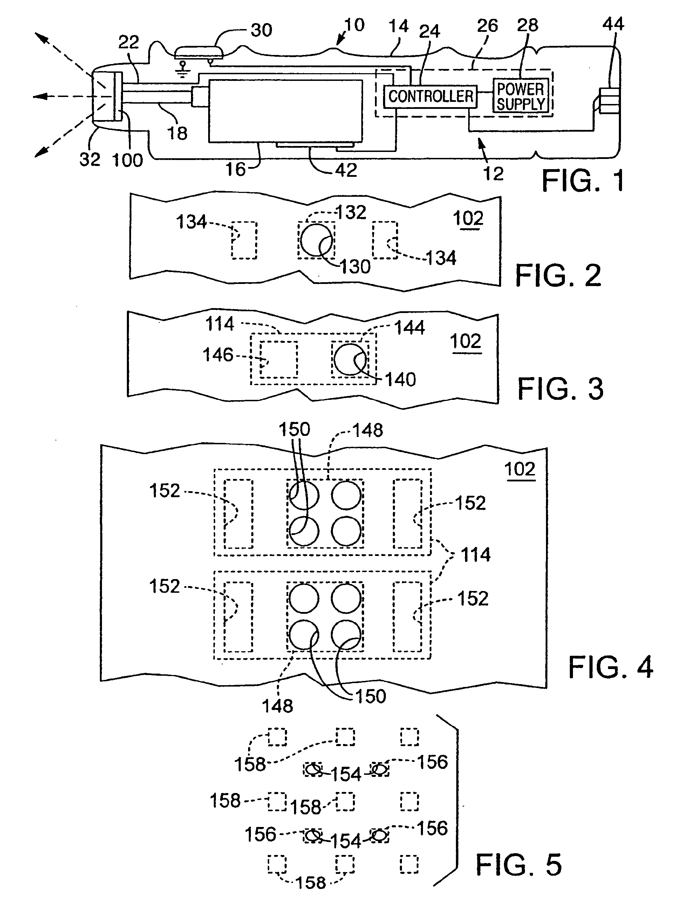

Fig. 2 is a top plan view of an isolated section of a single dual-inlet nozzle drop generator for use in a drop ejector device in an inhaler of the type shown in Fig. 1.
Fig. 3 is a top plan view of an isolated section of a single single-inlet nozzle drop generator for use in a drop ejector device in an inhaler of the type shown in Fig. 1.
Fig. 4 is a top plan view of an isolated section of two dual-inlet, four nozzle drop generators for use in a drop ejector device in an inhaler of the type shown in Fig. 1.

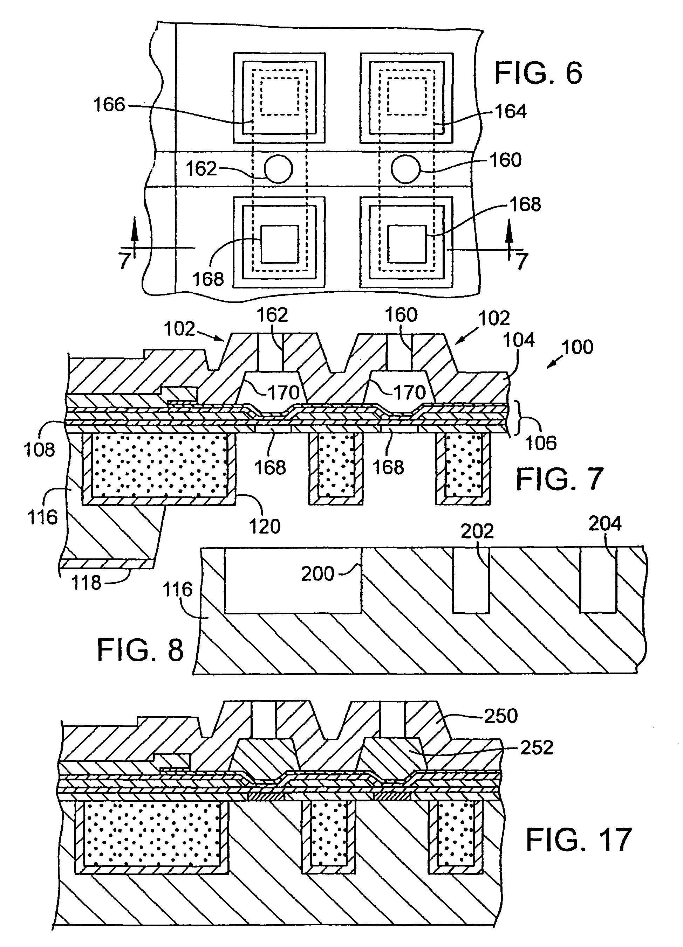

Fig. 5 is a top plan view of an isolated section of an array of nozzles drop generators exemplary of the type that may be used in a drop ejector device in an inhaler of the type shown in Fig. 1.
Fig. 6 is a top plan view of an isolated section of a drop generator head according to the present invention, and specifically illustrating a pair of single-nozzle, dual inlet drop generators.
Fig. 7 is a cross sectional view of taken along the line 7-7 of Fig. 6, illustrating a fully fabricated drop generator head according to the illustrated invention.
Figs. 8 through 17 comprise a sequential series of schematic cross section illustrations, each figure corresponding to the isolated section of the drop generator head illustrated in Fig. 6, and each illustrating in a schematic manner selected sequential steps in the fabrication of the drop generator head.
Fig. 8 illustrates the silicon substrate layer with trenches formed therein.

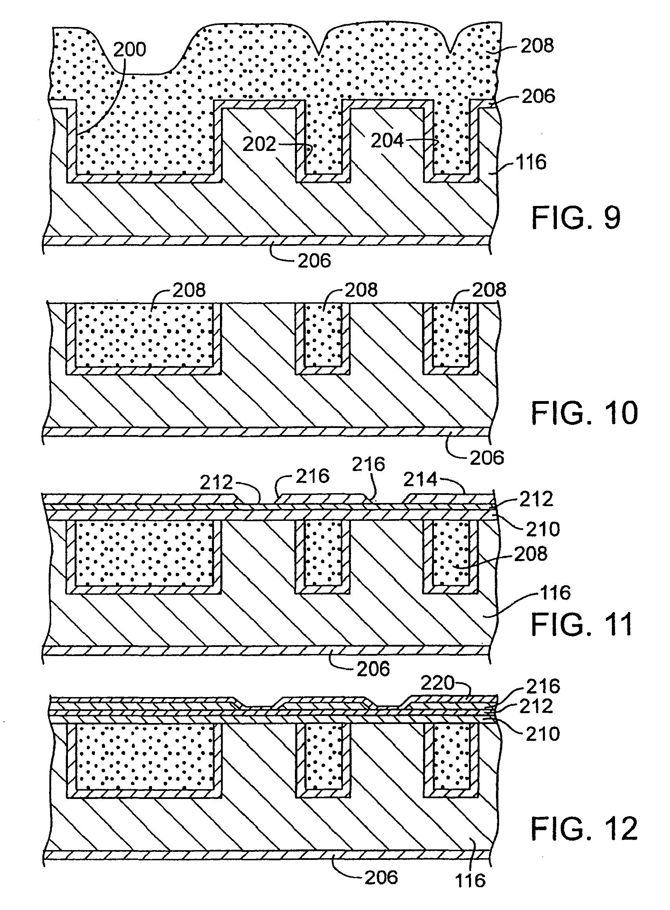

Fig. 9 illustrates silicon dioxide layers and polysilicon refill material.
Fig. 10 illustrates the head section shown in Fig. 9 after excess polysilicon refill material has been cleaned from it.
Fig. 11 illustrates a TEOS (tetraethylorthosilicate) layer deposited onto the head section shown in Fig. 10 with resistive layers and conductor layers.
Fig. 12 shows the section shown in the prior sequential figures after a passivation layer has been deposited.

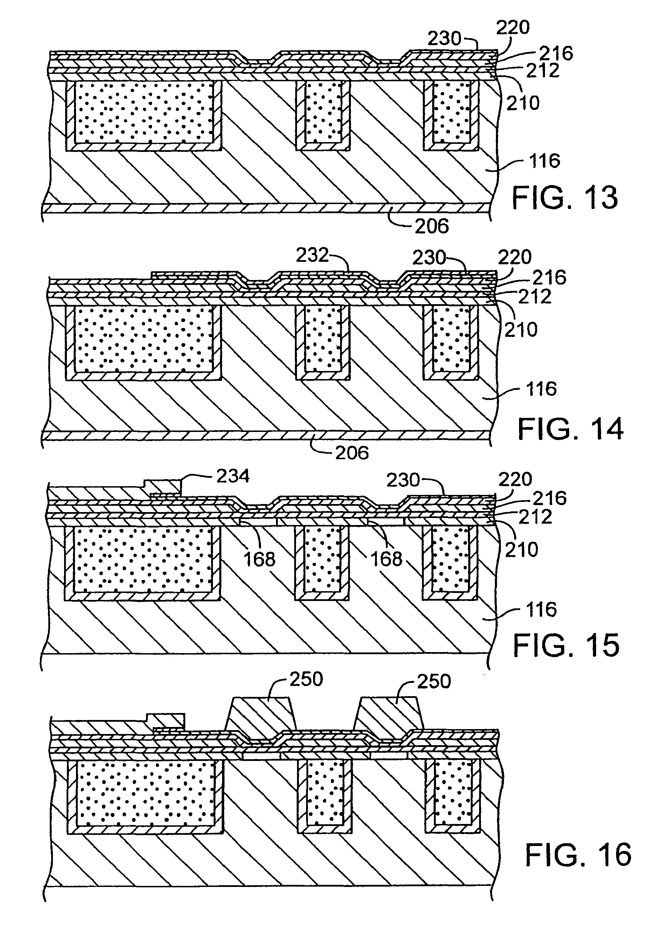

Fig. 13 illustrates the next sequential step in fabrication of the drop generator head, deposition of a tantalum layer.
Fig. 14 shows deposition of the next layer, titanium, in the sequential fabrication steps illustrated in Figs. 8 through 17.
Fig. 15 illustrates a sequential step in the formation of the nozzle orifices.
Fig. 16 shows formation of the fluid chambers.
Fig. 17 illustrates the drop generator head after deposition of low stress silicon dioxide (PECVD TEOS―ptasma enhanced chemical vapor deposition tetraethylorthosilicate) to complete the formation of the chambers.

### Detailed Description of Illustrated Embodiments

The device of the present invention is medicament aerosol generator or an inhalation system including a drop ejection device that receives fluid from a fluid supply system and a process for manufacturing the drop generator head in a manner to enhance the thermal efficiency of the drop ejection device. The drop ejection device is coupled to a controller and includes a plurality or multiplicity of individual drop generators, each of which ejects droplets of aerosolized fluid under the influence of the controller.

The inhalation system of the present invention includes circuitry that is electrically coupled to the drop generators and is configured to provide drop ejection pulses to each of the drop generators. By way of illustrative embodiment, drop ejection pulses are current or voltage pulses that are delivered to each of the drop generators. That they are delivered to each of the drop generators is to be understood to mean that they can be delivered to drop generators that are separately addressable or drop generators that are coupled in a parallel or serial arrangement wherein they are not individually addressable.

The inhalation system of the present invention also includes a fluid delivery system configured to deliver fluid to the drop generators at a controlled pressure level when measured adjacent the drop generators. Stated another way, the drop generators receive fluid having a controlled pressure level or range of pressure levels. As a result of the drop generator design and the characteristics of the fluid being delivered to the drop generators, the drop generators have a stable operating range that extends to gauge pressures below about -10 inches of water. Most preferably, the gauge pressure operating range is between about less than -10 inches of water to about -15 inches of water, but the drop generators have a stable operating range for gauge pressures anywhere in a range from below about -10 inches of water to about -45 inches of water.

As used herein, gauge pressure means the pressure difference between the pressure in question and outside atmospheric pressure. Gauge pressures for fluid drop generators are measured in inches of water (rather than mercury or PSI for example) because they tend to be of relatively low magnitude. References to a stable operating range, mean a range of pressures through which the drop generators can reliably eject drops without problems such as gulping in bubbles.

By way of illustrative embodiment, the drop generators are thermal type drop generators wherein each drop generator includes a nozzle or orifice that is disposed proximate to a current or voltage pulse activated resistor with supplied fluid therebetween. In response to receiving a pulse, the resistor generates a drive bubble in the fluid that forces ejection of an aerosol particle or droplet from the nozzle. The present invention is not limited to thermal drive bubbles, however, and includes designs that may incorporate piezo-activated drop generators.

By way of illustrative embodiment, the ejection device of the present invention includes at least 1000 fluid drop generators and preferably more than 9000 fluid drop generators. The circuitry delivers drop ejection pulses (meaning current or voltage or charge pulses) to each of the drop generators at a rate of at least 25 KHz and preferably at a frequency of at least 250 KHz.

Pulmonary drug delivery is most effective if the drop size is precisely controlled. Several physical characteristics of the droplets are important in providing effective pulmonary delivery so that medication delivered in the aerosolized droplets is quickly transferred into the blood stream. These include extremely small drop volume, preferably less than about 50 femtoliters and more preferably less than about 15 femtoliters, and a narrow range distribution of drop size, preferably between about 0.1 to 15 µm with a standard deviation of about 20%. Other characteristics of the inhalation system are similarly important, including a turn-on-energy (TOE) of about 0.014 µJ or less, a drop velocity of about 10 m per second or more as the droplets are expelled from nozzles, and a nozzle firing frequency of at least about 25 KHz, and more preferably about 250 KHz.

The present invention comprises an ejector head architecture capable of meeting these design criteria and additional criteria as detailed herein. The method of manufacturing the ejector head produces a highly thermally efficient device, which directly results in reduced energy consumption and better operating characteristics.

By way of background and to provide context, and with specific reference now to Fig. 1, the illustrated embodiment of the drop ejection device will be described as it is embodied in pharmaceutical delivery apparatus 10, which in this case is a handheld pulmonary delivery mechanism known as a metered dose inhaler (MDI) and is at times referred to herein as MDI 10. MDls such as the MDI 10 described herein are used for the delivery of aerosolized medications such as asthma medication and there are many variations of MDI delivery systems on the market. An MDI typically combines a drug with a propellant in a container that may be pressurized. The drug may be in the form of a liquid or a fine powder. Actuation of the device releases metered doses of aerosolized drug that is inhaled by the patient.

It will be appreciated that the MDI 10 illustrated in Fig. 1 is intended only to illustrate one of many possible pharmaceutical containers and delivery systems that may incorporate the a thermal-type drop generator as described herein. As used herein, the term "medication" is used generally to refer to any fluid or compound, whether biological, chemical or other, delivered to a patient, whether for treatment of a medical condition or some other purpose. Other common words may be used interchangeably, such as "pharmaceutical," or "medicament" or "bioactive agent" and similar words.

Before turning to a detailed description of the method for manufacturing the drop generator, the primary components of container 10 will be described generally with continuing reference to Fig 1.

Container 10 comprises an inhaler housing 14 that is configured to contain a reservoir or supply 16 of medication, which as noted is typically provided in liquid form, often as a solution. The medication supply 16 is coupled, as for example by a needle and septum interconnection or other airflow regulator such as a thermal resistive element or piezo element, to a conduit 18 in the housing 14 so that the medication in supply 16 is directed to a drop ejection device, illustrated schematically at 100 and described in detail below, that carries multiple drop generators and which is configured for generating appropriately sized aerosolized drops of the liquid from the medication supply 16. It will be appreciated that the illustration of Fig. 1 is schematic, and that an MDI must necessarily be designed to have the capability for the patient inhale a substantial volume of air with which the medication is mixed.

The drop ejection device 100 is electrically interconnected to a controller, shown at 24, which is part of the MDI control system 26, for example with a flex circuit 22. Among other functions described below, controller 24 generates and sends suitably conditioned control signals to drop ejection device 100 to initiate firing of nozzles and thus delivery of the medication. The MDI control system 26 includes controller 24, a power supply 28 (such as batteries) and operator switch 30. The controller 24 is an integrated circuit, typically in a CMOS chip that responds to the switch signal by directing to the drop ejection device 100 controlled current pulses for firing the drop generators as required. It will be appreciated that the control system can be configured in any of a number of ways and, most preferably, integrated with the housing 14 of the inhaler. Controller 24 includes appropriate processors and memory components. In some circumstances the integrated circuitry that defines controller 24 may be incorporated into a real time clock circuit, and vise versa.

In the case where MDI 10 is configured for delivery of medication via inhalation by the patient, the drop ejection device 100 is located near a mouthpiece or nosepiece 32. The drop ejection device 100 illustrated in Fig. 1 is thus located inwardly of the mouthpiece 32 to allow the aerosolized medication to mix with airflow. It will be understood that the control system 26 and the arrangement and orientation of the drop ejection device 100 in housing 14 provide for both precise metering of the amount of droplets ejected and of the amount of medication expelled, as well as the generation of suitably small droplets. That is, the expulsion of the medication from the medication supply 16 need not be accompanied with other mechanisms for reducing the volume of ejected liquid to suitably small droplets. The ejection route of medication aerosolized out of mouthpiece 32 is shown schematically with a series of arrows in Fig. 1.

MDI 10 may include a control sensor 42, which may be, for example, a temperature sensor operatively coupled to medication supply 16 so that the sensor is capable of detecting and monitoring the actual temperature of the medication contained within the supply reservoir 16. MDI 10 also preferably includes sensors such as appropriate circuitry in the drop ejection device 100 to monitor the pressure or the gauge pressure of fluid adjacent to the drop generators.

Suitable sensors 42 include integrated circuit temperature sensors such as thermisters and resistors, thin film metals, metal oxide semiconductor temperature sensors, CMOS or MOS transistors, bipolar transistors, circuits defining a Wheatstone bridge, and others. Suitable pressure sensors include transducers such as a piezo-electric device that generates a voltage in response to a pressure. Depending upon the specific usage, more than one sensor 42 and sensors of different types may be utilized.

Control system 26 includes a programming interface 44 connected to controller 24 and externally exposed at the rearward end of housing 14 for connection to an external computer. Programming interface 44 is a conventional interface that includes conductor pads 46 that interconnect the interface through traces (as in a flex circuit) and conventional buss interfaces to controller 24. The illustrated embodiment of programming interface 44 may be replaced, for example, with any suitable programming interface, including an infrared compliant data link, or other similar programming interface.

The mechanism for ejecting the liquid from the individual nozzles formed in drop ejection device 100 is by energizing heat transducers incorporated into the drop ejection device, as detailed below, to generate in liquid-filled chambers vapor bubbles. The heat-induced expansion of the liquid ejects the liquid through orifices.

Figs. 2 through 6 illustrate several nozzle arrangements or architectures that are suitable for use in fabricating a drop ejector device 100 according to the present invention.

Fig. 2 illustrates in a top plan view the arrangement of the orifice layer 102 having an orifice 130, heat transducer 132 that underlies the orifice 130, and inlets 134 disposed on either side of the orifice. Liquid flows from chamber 110 (Fig. 4) into inlets 134 and chamber 114, and is vaporized and ejected through orifice 130 by energizing heat transducer 132.

Fig. 3 shows in a top plan view similar to the view of Fig. 2 an alternative arrangement where a single orifice 140 is formed in orifice layer 102. A heat transducer 144 is positioned in control layer 106 as detailed above below orifice 140. Fluid flows into chamber 114 through a single inlet 146.

Fig. 4 shows in a top plan diagram yet another alternative arrangement of orifice, resistor, and inlet components of an exemplary pair of chambers 114 as formed in accordance with the present invention. In the embodiment illustrated in Fig. 4, a relatively large resistor 148 is used and the orifice layer 102 is formed with four orifices 150 overlying the four corner portions of the resistor. The liquid provided to the resistor 148 flows through a pair of inlets 152, one inlet on each side of the resistor 148.

Fig. 5 is a schematic diagram illustrating yet another one of several ways of arranging a small group of nozzles on an orifice layer. The diagram of Fig. 5 is a plan view wherein the orifices 154 are above the resistors 156. The resistors 156 are connected by the control layer 106 to the control system 126. In this embodiment, the inlets 158 are square in cross section and arranged so that there are at least two inlets 158 adjacent to each resistor 156.

Fig. 6 is yet another schematic diagram illustrating another one of several alternate arrangements of nozzles on an orifice layer. Two orifices 160 and 162 overlie resisters 164, 166, which are also referred to as heat transducers. Fluid is fed into the chambers through inlets such as inlets 168.

The spatial arrangement and relative positioning of the orifices and resistors shown in Figs. 2 through 6 are for illustrative purposes only and other arrangements are contemplated.

In all instances described above, the hydraulic diameter of the orifices (e.g., 104, 130, 140, 150, 154) is preferably between about 2.0µm and 4.0µm, and more preferably about 2.6µm. With this orifice size, the average droplet size expelled through each orifice is about 3µm.

From the foregoing discussion it will be appreciated that the drop ejection device 100 comprises a semiconductor die that incorporates thousands of nozzles such as nozzle 101. The nozzles may be of the types illustrated in Figs. 2 through 6, and the structure of drop ejection device 100 is detailed below.

### Method of Manufacture

An illustrated method of manufacturing drop ejection device 100 will now be described with reference to the illustrations of Figs. 7 through 17. It is to be understood that all of the drawings of Figs. 7 through 17 are highly schematic and essentially illustrate the section of drop ejection device 100 shown in Fig. 6, and the section thereof shown in Fig. 7.

With specific reference to the cross sectional view of Fig. 7, a schematic of a completely fabricated drop ejection device 100 is shown. A pair of drop generators 102 of the type that may be used in a drop ejection device 100 formed in accordance with one aspect of the present invention are shown. Drop generators 102 are shown in cross section. Although only two drop generators are shown in isolation in Fig. 7, it will be appreciated that the drop ejection device 100 comprises multiple thousands of drop generators in order to generate sufficient droplets in a given application.

With continued reference to Fig. 7 an orifice layer 104 is constructed having a pair of nozzles or orifices 102 defined in it. The orifice layer 104 overlies control layers shown generally at 106 that include resistive heat transducer elements 108 (also referred to herein as resisters), which are detailed below. Heat transducer elements 108 may further include circuitry and / or sensor capabilities to monitor gauge pressure at drop generators 102. Two inlets 168 (also referred to as "in feed holes" or "IFH") are defined in the control layers 106 to allow the liquid to flow into chambers 170, which define a small reservoir for holding liquid prior to ejection of the liquid from the chambers through the orifices 160. The control layers 106 overlie a solid substrate member 116 that has one side 118 in communication with fluid from, for example, supply 16, and which defines an inlet chamber 120 through which fluid flows into inlets 168. For reference purposes, the internal height dimension of chamber 170 is preferably about 2µm.

It will be appreciated that the word drop generator is used herein to describe generally the structures such as those shown in Fig. 7 for ejecting droplets, and, therefore, the word drop generator includes structures such as a nozzle or an orifice and a resister, and associated components.

The mechanism for ejecting the liquid from the chamber 170 is by energizing heat transducers164 to generate in the liquid-filled chamber a vapor bubble. Rapid expansion and vaporization of the bubble ejects the liquid through the orifice 162 in the form of small droplets. For computational purposes the heat transducer 164 is considered a planar member (such as a thin-film resistor) that, upon actuation heats the liquid in the chamber to very rapidly vaporize the liquid and thus eject it through the orifice in the form of a small droplet.

The series of drawing figures beginning with Fig. 8 and continuing through Fig. 17 detail a sequential series of the steps involved in fabricating the drop ejection device 100 shown in Fig. 7.

The solid substrate member 116 shown schematically in Fig. 8 represents the early stage of the fabrication process. Substrate member 116 is the base member on which the drop generators are formed. The solid substrate member 116 includes trenches 200, 202 and 204, which have been etched into member 116 using a silicon dry etch that preferably removes all overhangs that could form at the upper edges of the trenches. Process steps involved in forming the member 116 in Fig. 8 include 0.1 µm thermal Si02 growth, oxide dry etch, resist removal, Si dry etch of the trenches 200, 202 and 204 to a depth of approximately 10 µm, and oxide wet etch.

Fig. 9 illustrates 1µm thermal Si0₂ growth 206 on both sides of member 116, such that the layer 206 is found on the entire surface including the trenches 200, 202 and 204. Next, about 12µm of polysilicon 208 is deposited onto the upper surface of the member 116 ("upper" referring here to the orientation of member 116 in the figures). The polysilicon material fills trenches 200, 202 and 204. Depending upon the trench aspect ratio, it is possible that small voids may form in the polysilicon material deposited in the trenches, although no such voids are illustrated in Fig. 9.

The next illustrated sequential step in the fabrication process involves cleaning the upper surface of member 116 with poly CMP / Oxide CMP (Chemical Mechanical Polishing/Planarization) processing that exposes the original upper surface of member 116. The results of this processing step are shown in Fig. 10, where it may be seen that the polishing step has removed polysilicon material 208 from the upper surface of member 116, preferably resulting in the upper surface of member 116 being substantially planar and the trenches 200, 202 and 204 remaining filled with polysilicon 208.

With reference now to Fig. 11, the next sequential processing steps involve deposition of a TEOS (tetraethylorthosilicate) isolation layer 210, which preferably is about 8 µm in thickness. A combined resistor/conductor bilayer 212 is then deposited onto the isolation layer 210. The resistor layer 212 may be tantalum aluminum (TaAl) approximately 0.04 µm. An overlying conductor layer 214 is removed and a sloped conductor photo etch is performed prior to resistor etch to leave sloped sidewall portions 216 of overlying conductor layer 214 bordering the resistor layer 212 as illustrated.

The sloped conductor photo etch results in small resistor sizepreferably about 3µm by 3µm―having sloped upwardly facing sides, preferably without overhanging portions. The sloped conductor photo etch, and pre-resistor sputter etch improved the integrity of the passivation layer that is deposited onto the upper surface in the next process step, as detailed below. Moreover, the sloped sidewall portions 216 enhance the ability to deposit a thin passivation that remains undisrupted.

Next, with reference to Figs. 12 and 13, the passivation layer 220 and anticavitation layer 230 (Fig. 13), are deposited. Passivation layer 220 is preferably 1µm SiNx/0.1 SiCx, although other passivation materials may be used. The passivation layer 220 is dry etched with SiCx/SinX dry etc and resist removal. The combined passivation layer 220 is uninterrupted and provides important mechanical and electrical protection to the underlying resistor layer 212 and is preferably less than about 200 nm thick, and even more preferably about 100 nm thick. As used herein, the term "uninterrupted" means that the passivation layer 220 is substantially without voids or holes which would allow fluids to make contact with underlying layers such as the resister/conductor layer. The passivation layer is thus an effective barrier layer. With the passivation layer 220 having a thickness of < 200 nm, substantial improvements in thermal efficiency are obtained because more heat energy generated by the resistor 212 is conducted to the fluid being heated (as opposed to heating a relatively thicker passivation layer). Moreover, less residual heat remains in the drop generation device because less mass is heated in the passivation layer.

The reduced thickness of passivation layer 220 in turn translates directly into reduced energy requirements and allows the drop generation device to be reliably powered with batteries. Because less heating of the device occurs and less heat is retained, the incidence of "boiling" is reduced. As noted earlier, "boiling" occurs when the drop generation device is heated to the point that liquid medication boils even when no power is supplied to the resistive elements. This may happen, for example, when the substrate members and other structures become hot from extended energization of the resistive elements.

Turning next to Fig. 13, a 0.05µm layer 230 of Ta/1.2µm Au is deposited as a second conductor. The thin layer of tantalum serves as an adhesion and etchstop layer, and is conventionally left on top of passivated resistors as an anti-cavitation layer, though one embodiment of this more thermally efficient drop generator has the tantalum removed over the resistors at the end of the device fabrication, for additional improvement in thermal efficiency. Thinner tantalum increases thermal efficiency and the relatively thicker gold layer minimizes parasitic problems. The anticavitation layer is normally used in thermal generators of larger drops to provide further protection for the underlying passivation layer and resistor layers, preventing possible corruption of the passivation layers and subsequent damage to the resistor. The metal in the anticavitation layer resists cracking due to impact or stress, thereby assisting in preventing mechanical disruption of and damage to the passivation layer.

As previously noted, leaving the anti-cavitation layer 230 in place over the resistors is optional, but if it remains is preferably less than about 50 nm thick.

Fig. 14, illustrates the next step in the manufacturing process of depositing a 0.05µm thick layer of Ti, identified in Fig. 14 as layer 232.

Fig. 15 schematically represents deposition of a layer of SiNx/SiCx DSO, illustrated as layer 234. With continuing reference to Fig. 15, the resistor layer 206 has been removed from the lower side of member 116 to expose the silicon substrate. This prepares the member 116 for further processing steps during operation.

In Fig. 15, inlets 168 have been formed in isolation layer 210. These inlets 168 define infeed holes through which liquid medicament material flows into the drop generator heads.

The next sequential processing step is shown in Fig. 16. In this step 0.2µm Ti / 2µm AlCu is deposited to form the supporting matrices 250 upon which chambers 170 will eventually be formed. It will be appreciated that supporting matrices 250 will later be removed, after the orifice layer has been deposited, leaving voids (i.e., "chambers") where the supporting matrices 250 were deposited as shown in Fig. 16.

In one of the final processing steps, illustrated in Fig. 17, a 2µm layer of PECVD TEOS―in Fig. 17 labeled as layer 252, is deposited above the supporting matrices 250. Orifices 160 are formed in layer 252 above the matrices 250.

The member 116 is now ready for formation of the fluid pathways as shown in Fig. 7. This is accomplished by removing the silicon material that defines substrate member 116 with Si dry etching, to thereby define the inlet chambers 120 (see Fig. 7).

Finally, the supporting matrices 150 for chambers 170 are removed with lost wax etching to produce the finished drop generation device illustrated in Fig. 7.

Table 1 provides selected design specification criteria for drop generator 100.

**Table 1.**

| Design Specification Criteria | Value |
|---|---|
| Passivation Layer Thickness | < 200 nm, preferably 100 nm |
| Anticavitation Layer Thickness | < 50 nm (optional layer) |
| Turn on energy (µJ) | 0.011 - 0.017¹ |
| Drop velocity (m/s) | > 10 |
| Drop volume (fL) | < 15 |
| Drop size (µm) | < 3µm |
| Firing Frequency (KHz) | > 25, preferably about 200 |
| Nozzle Density (nozzles / mm²) | > 100, preferably > 250 |
| Drop Generation Rate (drops per second) | >1 x 10⁹, preferably 1.8 x 10⁹ |
| Number of Nozzles/drop ejector device | >4,000, preferably 9000 |

| | |
|---|---|
| Notes: 1. The turn on energy varies depending upon the nozzle architecture as shown in Figs. 2 through 6. | |

Firing drop generator 100 described herein having 9000 nozzles at a frequency of 200 KHz results in the generation of 1.8 billion droplets per second. For purposes herein, flux or total particle flux refers to the number of droplets ejected per unit time from the drop ejection device 100. A greater number of nozzles firing simultaneously increases the flux. Suitable flux is attained with a drop generator having at least about 4000 nozzles firing at a frequency of at least about 100 KHz. A drop generator operating within these constraints provides for accurate dosage control and delivery of medication in a handheld MDI 10.

When the TOE is in the range specified in Table 1, a standard power supply 28 such as batteries configured for use in an MDI 10 provides sufficient battery life.

The fluid characteristics of medication delivered to drop generator 100 can have significant impact on the performance of the MDI 10 and the droplets delivered through it. For example, an exemplary range of fluid medications for delivery through nozzle generator 100 have surface tensions in a range of about 20 to 70 dynes/cm². In a drop ejection device 100 of the type disclosed herein, an acceptable gauge pressure operating range for effective drop generator 101 operation preferably extends below about -10 inches of water (for medications having surface tension in the range noted above, 20 to 70 dynes/cm²) measured proximate to the drop generator 101. Tests have shown that with medication having a surface tension at or near the low end of this range, 20 dynes/cm², and with a nozzle orifice size of about 3.0µm, a gauge pressure operating range of about -13 inches of water is achieved. As the nozzle orifice size decreases, the effective gauge pressure operating range increases. With medication having a surface tension at or near the upper end of the range noted, 70 dynes/cm², and with a nozzle orifice size of about 3.0µm, a gauge pressure operating range of about -45 inches of water is achieved.

When drop generator 100 thus is fabricated with orifice architectures of the type described above, the drop generators 101 are operable with a gauge pressure below about -10 inches of water, even with medications having a surface tension as low as 20 dynes/cm². Having a broad acceptable gauge pressure range has significant beneficial effects on the reliability of the MDI 10. For example, the drop generator is very shock resistant and the occurrence of shock depriming of the drop generators and bubble ingestion is substantially reduced. The drop generators are also operable under a wide range of medication fluid characteristics such as surface tension, and delivery pressures.

Having here described illustrated embodiments of the invention, it is anticipated that other modifications may be made thereto within the scope of the invention by those of ordinary skill in the art. It will thus be appreciated and understood that the spirit and scope of the invention is not limited to those embodiments, but extend to the various modifications and equivalents as defined in the appended claims.

## Claims

1. A drop ejection device, comprising:
a substrate member (116) having a heat transducer (212);
an orifice layer (102) attached to the substrate member (116) and having an orifice (162) formed therethrough to define a chamber (170) adjacent the heat transducer;
a fluid inlet (168) in the substrate member (116) to define a fluid channel from a supply of fluid into the chamber (170);
an uninterrupted passivation layer (220) over the heat transducer (212), the passivation layer (220) less than about 200 nm thick.

2. The drop ejection device according to claim 1 further comprising said passivation layer (220) about 100 nm thick.

3. The drop ejection device according to claim 1 further comprising an anti-cavitation layer (230) on said passivation layer.

4. The drop ejection device according to claim 3 wherein said anti-cavitation layer (230) comprises at least tantalum.

5. The drop ejection device according to claim 1 further comprising at least 1000 drop generators (101).

6. A method of manufacturing a drop ejection device, comprising the steps of:
(a) providing a substrate (116) having a heat transducer layer (212);
(b) depositing an uninterrupted passivation layer (220) on the heat transducer layer (212), the passivation layer (220) less than about 200 nm thick.

7. The method of claim 6 including the stop of depositing an anti-cavitation layer (230) on the passivation layer (220), said anti-cavitation layer (230) defined by at least tantalum.

8. The method of claim 7 wherein said anti-cavitation layer (230) deposited on said passivation layer (220) is no more than about 0.05 µm thick.

9. The method of claim 7 including the step of forming orifices (162) in said drop ejection device to thereby define drop generators (101).

10. The method of claim 9 including the step of forming orifices (162) in said drop ejection device at a density of at least about 250 orifices per square millimeter.
